Europäisches Patentamt

European Patent Office

Office européen des brevets

(19)

(11) Veröffentlichungsnummer: **0 194 502**
**B1**

(12) **EUROPÄISCHE PATENTSCHRIFT**

(45) Veröffentlichungstag der Patentschrift: **25.04.90**

(51) Int. Cl.⁵: **G 01 N 33/52**

(21) Anmeldenummer: **86102433.9**

(22) Anmeldetag: **25.02.86**

(54) **Mittel und Verfahren zur Abtrennung von Plasma oder Serum aus Vollblut.**

(30) Priorität: **09.03.85 DE 3508427**

(43) Veröffentlichungstag der Anmeldung:
**17.09.86 Patentblatt 86/38**

(45) Bekanntmachung des Hinweises auf die
Patenterteilung:
**25.04.90 Patentblatt 90/17**

(84) Benannte Vertragsstaaten:
**BE CH DE FR GB IT LI NL SE**

(56) Entgegenhaltungen:
**EP-A-0 045 476**
**EP-A-0 057 110**
**EP-A-0 183 991**
**FR-A-2 448 151**
**US-A-3 552 928**

(73) Patentinhaber: **MERCK PATENT GESELLSCHAFT
MIT BESCHRÄNKTER HAFTUNG
Frankfurter Strasse 250 Postfach 4119
D-6100 Darmstadt (DE)**

(72) Erfinder: **Limbach, Berthold, Dr.
Otto-Hahn-Strasse 8
D-6104 Seeheim-Jugenheim (DE)**
Erfinder: **Helger, Roland, Dr.
Ludwigshöhstrasse 85
D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Mittel und Verfahren zur einfachen Abtrennung von Plasma oder Serum aus Vollblut.

Die Gewinnung von Plasma oder Serum zum Einsatz als Probenmaterial für klinisch-chemische Untersuchungen ist von entscheidender Bedeutung, da nur auf diese Weise ein störungsfreie Analyse gewährleistet werden kann. Die dabei erforderliche Abtrennung der Erythrozyten erfolgt üblicherweise durch Zentrifugation der zu untersuchenden Vollblutproben. Dieses Verfahren ist bei der Durchführung größerer Testprofile die Methode der Wahl, andererseits erweisen sich der apparative Aufwand und die erforderlichen größeren Probevolumina bei geringer Analysenzahl als nachteilig. Insbesondere gilt dies für den Bereich der Schnelltests. Unter diesem Begriff versteht man Systeme, die aus einem saugfähigen, mit Reagenzien getränkten Träger bestehen. Auf diesen wird eine geringe Menge der zu untersuchenden Lösung zumeist ohne weitere Verdünnung oder Lösungsmittelzugabe aufgegeben, um anschließend die jeweilige ablaufende Bestimmungsreaktion visuell oder reflekto metrisch auszuwerten. Es ist leicht ersichtlich, daß bei solchen Systemen eine zusätzliche Probenaufbereitung, etwa durch eine Zentrifugation, den Zeitaufwand für die einzelnen Analysen wesentlich erhöht. Zudem wäre der Einsatz von Kapillarblut wegen der für die Zentrifugation erforderlichen größeren Probevolumina nicht möglich. Auf der anderen Seite ist eine saubere Abtrennung der Erythrozyten unbedingt erforderlich, weil sonst Färbungen oder Trübungen das Analysenresultat beeinflussen oder eine Auswertung völlig unmöglich machen würden.

Da die Bestimmung von klinisch-chemischen Parametern im Vollblut wesentlich aussagekräftiger ist als die von Urinproben erhaltenen Resultate, war man bestrebt, Schnelltests für die Vollblutanalyse zu entwickeln. Die ersten Versuche, ein auf Schnelltests adaptierbares Auftrennverfahren zu erhalten, bestanden darin, die Oberfläche des Trägermaterials so zu verändern, daß die im Blut zu bestimmenden Substanzen noch in den Träger diffundieren können, die Erythrozyten aber zurückgehalten werden (Erythrozytenbarriere). Als geeignete Substanzen fanden dabei u.a. Aminosäuren oder deren Amide, Öle, Paraffine, Silikone oder Cholesterin Verwendung. In ähnlicher Weise ist es auch möglich, das Trägermaterial mit semipermeablen Membranen zu überziehen (US 3092465), um einen Ausschluß der roten Blutkörperchen zu erreichen. Prinzipiell lassen sich mit Hilfe dieser Verfahren für einige Tests funktionsfähige Systeme entwickeln, doch sind neben der Reproduzierbarkeit, der Herstellung und der Handhabung (Abwaschen des Blutes erforderlich), die geringe Anzahl möglicher Tests (nur niedermolekulare Bestandteile des Vollblutes) nachteilig. Höhermolekulare Substanzen wie Proteine oder Lipide können auf diese Weise nicht bestimmt werden, weil sie ebenfalls durch die Barriere zurückgehalten werden.

Weiterhin ist bekannt, die Vollblutprobe zur Abtrennung der Erythrozyten durch eine spezielle Abtrennschicht diffundieren zu lassen. Diese Schicht kann aus einem Membranfilter, anderen Materialien mit Filtereigenschaften oder aus Glasfasern (EP 45476) bestehen. Gleichfalls möglich sind Gele bestimmter Porengröße (DE 3003189). Dimensionierung und Anordnung dieser Materialien können dabei veriiert werden. Bei diesen Systemen könnnen neben Schwierigkeiten bei der Diffusion bestimmter hochmolekularer Substanzen Probleme mit der Menge der durchdiffundierten Flüssigkeit sowie dem Zeitbedarf für diesen Prozeß auftreten.

Eine weitere Möglichkeit, Serum oder Plasma herzustellen, besteht darin, die Erythrozyten durch Zusatz bestimmter Stoffe, z. B. Lektine, zu agglutinieren. Üblicherweise geschieht dies dadurch, daß die zu untersuchende Blutprobe in einem Gefäß mit dem entsprechenden Lektin oder Lektingemisch versetzt wird und der das Serum oder Plasma enthaltende Überstand nach Absetzen der Erythrozyten entfernt wird. Das Gefäß ist dabei am einfachsten das Blutentnahmerohr selbst oder ein übliches Probengefäß.

In der EP 57110 ist ein Reaktionskörper mit zwei Kammern beschrieben, wobei eine Kammer als Blutauftrennbereich Verwendung finden kann. Die Auftrennung erfolgt über Latex-Beads, an welche Lektine immobilisiert sind. Der Transport der Flüssigkeit nach erfolgter Auftrennung geschieht durch Anlegen eines äußeren Drucks. Dieses als theoretisches Beispiel dargelegte Funktionsprinzip erfordert wegen seiner Komplexität eine mechanisierte Durchführung; zudem ist die Verwendung immobilisierter Lektine in Bezug auf Bindungskapazität und Verwendungsfähigkeit der einzelnen Lektine problematisch.

Aus US—A—3 552 928 ist eine saugfähige Matrix bekannt, die zur Abtrennung von Plasma oder Serum aus Vollblut mit wasserlöslichen Aminosäuren imprägniert ist. Bedingt durch die hohe Konzentration an Aminosäuren (die Tränklösung enthält 2—50 Gew.%) werden diese während des Abtrennvorgangs in die zu analysierende Lösung überführt und können dort das Analysenergebnis verfälschen.

In der EP—A—183 991 ist ein Verfahren beschrieben, bei dem eine mit Lektin imprägnierte Matrix nach Aufgabe einer Vollblutprobe mit einem Verdünnungspuffer mehrfach durchspült wird. Man erhält auf diese Weise verdünntes Plasma oder Serum, in dem die eigentliche Bestimmungsreaktion durchgeführt werden kann. Das Volumen des verdünnten Plasmas oder Serums liegt dabei in der Größenordnung von 1 ml. Ein solches Verfahren ermöglicht zwar die photometrische Bestimmung von Substanzen im Vollblut, ist aber in seiner Dimensionierung für die üblichen Schnelltestsysteme, die mit Volumina < 100 µl arbeiten, nicht geeignet. Auch ist der erforderliche mehrfache Durchspülungsprozeß für solche Systeme nicht praktikabel.

Der Erfindung liegt die Aufgabe zugrunde, ein einfaches Verfahren zur Abtrennung von Plasma oder Serum aus Vollblut zu finden, mit dem kleine Blutvolumina schnell aufgetrennt werden können.

EP 0 194 502 B1

Es wurde gefunden, daß eine Abtrennung von Plasma oder Serum aus Vollblut schnell und einfach durchgeführt werden kann, wenn man die Blutprobe auf eine saugfähige Matrix aufbringt, die zuvor mit einem Lektin oder Lektingemisch imprägniert wurde und anschließend durch Anwenden eines externen Druckes das gebildete Plasma oder Serum z.B. in eine die Testreagenzien enthaltende weitere Schicht überführt.

Gegenstand der Erfindung ist ein Verfahren zur Abtrennung von Plasma oder Serum aus Vollblut durch Aufbringen der Vollblutprobe auf eine aus einer saugfähigen, mit Lektin imprägnierten Matrix bestehende Abtrennschicht, das dadurch gekennzeichnet ist, daß man nach einer vorgegebenen Zeit durch Anwendung eines Drucks das gebildete Plasma oder Serum in weitere, mit Reagenzien imprägnierte Schichten überführt, wobei das Volumen der zu trennenden Vollblutprobe maximal 50%, vorzugsweise 20 bis 30% des Saugvolumens der Abtrennschicht beträgt.

Überraschenderweise wurde gefunden, daß mit Hilfe einer mit Lektin oder einem Lektingemisch imprägnierten saugfähigen Matrix aus einem Textilgewebe oder einem zellulosehaltigem Material eine schnelle und einfache Auftrennung von Vollblut in Plasma oder Serum ohne zusätzliche Verdünnung und ohne Durchspülzprozesse möglich ist. Diese Tatsache muß als umso überraschender angesehen werden, als die verwendete Matrix keine Filter- bzw. Ausschlußeigenschaften besitzt, wie beispielsweise Glasfasern, und die verwendeten Lektine lediglich aufgetrocknet werden, ohne daß eine kovalente Bindung erforderlich ist. Die Vollblutprobe wird dabei auf die imprägnierte Matrix aufgegeben und nach einer vorgegebenen Zeit das gebildete Plasma oder Serum durch Anlegen eines äußeren Drucks in einer oder mehreren weiteren Schichten der Bestimmungsreaktion zugeführt. Als Matrix können dabei allgemein alle saugfähigen Materialien mit Gewebe- oder Faserstruktur Verwendung finden, die keine Filter- oder Ausschlußeigenschaften für hochmolekulare Stoffe wie z. B. Proteine, Lipide oder Blutzellen (Erythrozyten, Leukozyten) zeigen. Als besonders vorteilhafte Materialien haben sich Baumwolle- und Viskosegewebe sowie diese Matrices enthaltende Mischgewebe und Zellulosematerialien herausgestellt.

Eine besondere Bedeutung kommt dabei dem Saugvermögen des verwendeten Material zu. Eine Matrix besitzt im Sinne der Erfindung dann ein genügend großes Saugvermögen, wenn eine innerhalb der maximal aufsaugbaren Flüssigkeitsmenge liegendes Flüssigkeitsvolumen in maximal 10 bis 15 Sekunden vollständig aufgesaugt wird.

Die Dicke der Abtrennschicht ist sowohl vom Saugvermögen und der Trennkapazität der verwendeten Matrix als auch vom Volumen der aufzutrennenden Blutprobe abhängig. Das Saugvolumen der Abtrennschicht sollte geringer als 50%, vorzugsweise etwa 20—30% des Gesamtsaugvolumens der Abtrennschicht sein. Hat eine bestimmte Matrix eine kleinere Dicke als erforderlich, können z. B. mehrere Lagen derselben oder verschiedener Materialien übereinander gelegt werden und als Abtrennschicht fungieren.

Als Lektine können alle Substanzen eingesetzt werden, die eine Agglutination der Erythrozyten bewirken. Geeignete Lektine sind z. B. solche aus Solanum tuberosum, Tritium vulgaris, Ricinus communis, Sojabohnen, Phaseolus vulgaris, Lentil, Weizenkeimen, Phytolacca americana. Ein Zusatz von die Agglutination verstärkender Substanzen, z. B. kationische Polymere, wie Hexadimethrinbromid, ein Polymer aus N,N,N',N'-Tetramethylhexamethylendiamin und Trimethylenbromid; N,N,N',N'-Tetramethyl-1,6-hexandiamin-Polymer mit 1,3-Dibrompropan; Poly(N,N,N',N'-Tetramethyl-N-trimethylenhexamethylendiammoniumdibromid) ist ebenfalls möglich.

Die Imprägnierung der Matrix mit dem Lektin bzw. Lektingemisch erfolgt üblicherweise durch Tränken mit einer entsprechenden wässrigen Lösung und anschließendes Trocknen. Dabei kann die Tränklösung neben dem Lektingemisch und den Puffersubstanzen zusätzliche Reagenzien enthalten, die etwa die Hämolyse der Erythrozyten verhindern, die Agglutination verstärken oder die Gerinnung hemmen. Von Fall zu Fall sind die Konzentrationen der Lektine wie der zusätzlichen Substanzen zu optimieren. Im allgemeinen werden dabei isotonische Lösungen im pH-Bereich zwischen 6,0 und 9,0 bevorzugt.

Die Überführung des gebildeten Plasmas oder Serums in die weiteren Schichten durch Anlegen eines äußeren Drucks kann z. B. durch Aufdrücken eines Stempels oder eines beliebig gearteten, an der Unterseite glatten, stabilen Gegenstandes erfolgen. Darüber hinaus sind auch nicht mechanische Druckanwendungen, etwa durch Anlegen eines Über- oder Unterdrucks, möglich.

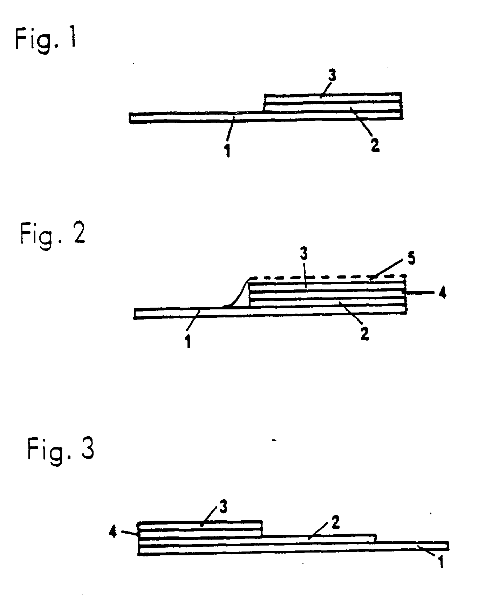

Die Trennmatrix wird vorzugsweise in Verbindung mit diagnostischen Mitteln eingesetzt. Ein solches diagnostisches Mittel besteht im einfachsten Fall (Fig. 1) aus einer steifen Unterlage (1), auf die eine Reaktionsschicht (2) und die Abtrennschicht (3) aufgebracht sind. Die Reaktionsschicht besteht dabei aus einer mit den für eine Nachweisreaktion erforderlichen Reagenzien imprägnierten saugfähigen Matrix z. B. aus Filterpapier oder Kunststoffilmen. Entsprechende Materialien und die Methoden zu ihrer Imprägnierung sind aus dem Stand der Technik bekannt. Als Unterlage kann prinzipiell jedes stabile Trägermaterial benutzt werden, vorzugsweise transparente Materialen wie Kunststofffolien oder Glasplatten. Das Unterteil dieser Unterlage kann als Griff ausgebildet sein.

Zur Testdurchführung wird ein Blutstropfen auf die Trennschicht aufgegeben und diese nach wenigen Sekunden durch vorsichtiges Pressen mit einem Stempel oder einem ähnlichen Gegenstand auf die Reaktionsschicht aufgepreßt. Dieser Vorgang kann mechanisiert oder teilmechanisiert durchgeführt werden, etwa durch Durchziehen des Schnelldiagnostikums durch ein Paar gegeneinanderlaufender Walzen. Bei diesem Prozeß wird das in der Trennschicht gebildete Plasma oder Serum mit der Reaktionsschicht in Verbindung gebracht und die Bestimmungsreaktion gestartet. Besonders vorteilhaft ist dabei, daß die Zeitspanne vor dem Überführungsprozeß, in der die Auftrennung des Vollblutes erfolgt, variabel von einigen Sekunden bis zu mehreren Minuten gewählt werden kann. Dies ermöglicht bei der manuellen Durchführung die Unabhängigkeit von einem festen Zeitprogramm, andererseits ist bei einer mechanisierten Durchführung eine Vorbehandlung der Probe vor der eigentlichen Bestimmungsreaktion (z. B. Vortemperierung oder Vorreaktion) möglich. Nach Ablauf einer festgelegten Reaktionszeit erfolgt die Auswertung visuell oder reflektometrisch entweder von der Unterseite her durch die transparente Unterlage oder nach Abziehen der Trennschicht durch eine geeignete Vorrichtung von der Oberseite des Schnelldiagnostikums her.

Einen weiteren möglichen Aufbau des erfindungsgemäßen Schnelltests zeigt Fig. 2, wobei zusätzlich zu dem zuvor beschriebenen Aufbau zwischen Abtrennschicht und Reaktionsschicht eine hydrophobe Zwischenschicht (4) eingearbeitet ist. Diese kann aus einem Netz eines hydrophoben Kunststoffs oder aus einem hydrophobierten grobmaschig gewebten Textilstoff bestehen. Entsprechende Verfahren zum Hydrophobieren von Geweben sind bekannt. Die Zwischenschicht fungiert als zusätzliche Barriere für die Erythrozyten, zudem läßt sie den Flüssigkeitskontakt erst bei stärkerem Anpressen eintreten. Darüber hinaus kann zum Schutz der Trennschicht eine die Probeflüssigkeit nicht zurückhaltende Abdeckschicht (5) aufgebracht sein. Die Durchführung und Auswertung bei dieser Schnelltestanordnung erfolgt wie oben beschrieben.

Weiterhin ist ein Aufbau möglich, bei dem die Trennschicht (3) und — falls vorhanden — die Zwischenschicht (4) eine kleinere Fläche einnehmen als die Reaktionsschicht (2) (Fig. 3). In diesem Fall kann das aufgetrennte Plasma oder Serum in den nach oben hin unbedeckten Teil der Reaktionsschicht diffundieren, so daß eine Auswertung von der Oberseite des Schnelldiagnostikums möglich ist.

## Beispiel 1

### Vollblutauftrenneigenschaften verschiedener Systeme

Verschiedene Matrices wurden mit den im folgenden aufgeführten Lektingemischen getränkt und bei 20—30°C getrocknet. Folgende Materialien und Lösungen kamen dabei zum Einsatz:

Matrix 1: 3 Lagen eines Baumwollgewebes mit Flächengewicht 120 [g/m²] und Fadendichte [Fädern/cm] 25,0 (Kette), 18,7 (Schuß) sowie Garnfeinheit [tex] 16,2 (Kette), 34,1 (Schuß)

Matrix 2: 2 Lagen eines Baumwollgewebes mit Flächengewicht 123 und Fadendichte 17,9 (Kette), 11,3 (Schuß) sowie der Garnfeinheit 24,8 (Kette), 67,0 (Schuß)

Matrix 3: 1 Lage eines Baumwollgewebes mit Flächengewicht 316 und Fadendichte 13,6, 13,6 und 19,9 sowie der Garnefeinheit 48,0, 24,2 und 29,9

Matrix 4: 2 Lagen eines Mischgewebes aus 70% Polyester und 30% Viskose mit einem Flächengewicht von 224 und der Fadendichte von 12,3 (Kette), 11,8 (Schuß) sowie der Garnfeinheit von 87,2 (Kette), 88,2 (Schuß)

Matrix 5: 2 Lagen eines Mischgewebes aus 56% Polyester und 44% Wolle mit einem Flächengewicht von 187 und der Fadendichte von 31,9 (Kette), 34 (Schuß) sowie der Garnfeinheit von 2 × 12,4 (Kette), 24,8 (Schuß)

Matrix 6: 3 Lagen eines Wollgewebes mit Flächengewicht 113 und der Fadendichte 25,9 (Kette), 22,8 (Schuß) sowie der Garnfeinheit 19,5 (Kette), 19,4 (Schuß)

Matrix 7: Verbandszellstoff, DIN A 19310 Flächengewicht 124

Lektingemische:

In 0,006 mol/l Phosphatpuffer, pH 7,0 mit 0,02 mol/l Natriumchlorid und 1,4% kationischem Polymer waren zusätzlich jeweils enthalten:

Lektingemisch 1: 200 µg/ml Lektin aus Sojabohnen

Lektingemisch 2: 200 µg/ml Lektin aus Ricinus communis

Lektingemisch 3: 400 µg/ml Lektin aus Weizenkeimen und 200 µg/ml Lektin aus Phytolacca americana

Lektingemisch 4: 200 µg/ml Lektin aus Sojabohnen und 150 µg/ml Lektin aus Solanum tuberosum

Lektingemisch 5: 150 µg/ml Lektin aus Weizenkeimen und 300 µg/ml Lektin aus Sojabohnen und 500 µg/ml Lektin aus Phaseolus vulgaris

Aus den einzelnen imprägnierten Matrices wurden 10 × 30 mm große Rechtecke ausgeschnitten und auf gleichgroße Rechtecke aus Filterpapier 25 SS 81 der Firma Schleicher und Schüll aufgelegt. Zur Ermittlung der Vollblutauftrennung wurden an einem Ende der Streifen jeweils in der Mitte der Abtrennschicht 50 µl Vollblut aufgegeben und nach einigen Sekunden die Abtrennschicht mit Hilfe eines Stempels vorsichtig auf die Filterpapierschicht gedrückt. Nach Abheben der Trennschicht zeigte die Benetzung des Filterpapiers mit einer farblosen bis schwach gelblichen Lösung eine gute Auftrennung an; bei einer schlechten Auftrennung werden hingegen durch Hämolyse oder nicht abgetrennte Erythrozyten zusätzlich rot gefärbte Punkte oder Bereiche bis hin zur vollständigen Rotfärbung des benetzten Bereichs erhalten. Solche unbrauchbaren Resultate sind in der folgenden Tabelle mit "−" gekennzeichnet, gute Ergebnisse mit "+" und sehr gute Ergebnisse mit "++".

4

TABELLE 1
Eignung verschiedener Matrices und Lektingemische

| Matrix | Lektingemisch | Ergebnis der Vollblutauftrennung | |
|---|---|---|---|
| | | mit Lektingemisch | ohne Lektingemisch |
| 1 | 1 | ++ | − |
| 1 | 2 | ++ | − |
| 1 | 3 | ++ | − |
| 1 | 4 | ++ | − |
| 1 | 5 | ++ | − |
| 2 | 2 | ++ | − |
| 3 | 2 | + | − |
| 4 | 1 | + | − |
| 5 | 3 | ++ | − |
| 6 | 4 | + | − |
| 7 | 5 | ++ | − |

Insgesamt wurden mit den aufgeführten Matrices und Lektingemischen gute bis sehr gute Auftrennergebnisse erhalten; die Vergleichsversuche ohne Imprägnierung mit Lektingemisch zeigen dagegen unbrauchbare Resultate.

Beispiel 2

Einfluß der Zwischenschicht auf die Vollblutauftrennung

Zur Herstellung einer Zwischenschicht wurde eine Lage des Gewebes von Matrix 1 mit einer mit Paraffin gesättigten Lösung von Dichlormethan versetzt und anschließend getrocknet. Dann wurden analog zu Beispiel 1 Trennsysteme unter Verwendung von Matrix 1 und Lektingemisch 3 hergestellt. Parallel hierzu wurden bei Systemen mit Zwischenschicht zwischen Trennschicht und Filterpapier die vorher hergestellte, auf gleiche Größe geschnittene Zwischenschicht eingefügt. Auf beide Systeme wurde dann analog zu Beispiel 1 50 µl Vollblut aufgegeben und die Trennschicht mit unterschiedlichem Druck auf das Filterpapier gepreßt. Dabei wurden folgende Resultate erhalten:

| Anpreßdruck | Ergebnis der Vollblutauftrennung | |
|---|---|---|
| | System ohne Zwischenschicht | System mit Zwischenschicht |
| gering | + | unvollständige Trankung des Filterpapiers |
| mittel | +/− | + |
| mäßig | − | + |
| groß | − | − |

Bei beiden Systemen können gute Auftrennergebnisse erhalten werden, allerdings ist das System mit Zwischenschicht weniger anfällig bei Schwankungen im Anpreßdruck.

EP 0 194 502 B1

## Beispiel 3

Bestimmung von Glucose im Vollblut

Matrix 1 wurde mit folgender Lösung getränkt und anschließend getrocknet:

2,6 mg Lektin aus Sojabohnen und

1,0 mg Lektin aus Ricinus communis

11,7 mg Natriumchlorid und

140 mg kationisches Polymer in

10 ml 0,01 mol/l Phosphatpuffer, pH 7,0

Die imprägnierte Matrix wurde zur Herstellung der Trennschicht in jeweils 10 × 30 mm große Rechtecke geschnitten. Danach wurde ein Filterpapier (25 SS 81 von Schleicher und Schüll) mit einer Lösung von

55 mmol/l NAD$^+$

2 KU/l Glucosedehydrogenase

2,4 mmol/l 3-(4,5-Dimethylthiazol-2-yl)-2,5-diphenyl-2H-tetrazoliumbromid

20 kU/l Diaphorase in

0,05 mol/l Phosphatpuffer, pH 7,6 enthaltend 2% EDTA getränkt, getrocknet und gleichfalls in 10 × 30 mm große Rechtecke geschnitten.

Zur Herstellung eines Systems zur Bestimmung von Glucose im Vollblut wurde auf einen Objektträger aus Glas jeweils ein Rechteck Filterpapier und eine Trennschicht aufgebracht. Auf die Oberseite der Trennschicht wurden dann jeweils 50 μl Vollblut unterschiedlichen Glucosegehaltes aufgegeben und nach 15 Sekunden die Trennschicht auf die imprägnierte Filterpapierschicht so aufgedrückt, daß das Filterpapier unterhalb der Blutaufgabestelle gleichmäßig benetzt wurde. Anschließend wurde von der Unterseite des Systems durch den Objektträger hindurch der Reaktionsablauf beobachtet. Innerhalb von einer Minute bildete sich in dem von Flüssigkeit benetzten Bereich eine Blaufärbung, die derjenigen entsprach, die man erhielt, wenn man anstelle des Blutes abzentrifugiertes Plasma des gleichen Blutes einsetzte.

## Beispiel 4

Bestimmung von γ-Glutamyltranspeptidase im Vollblut

Die Abtrennschicht wurde wie in Beispiel 1 hergestellt. Zur Herstellung der Reaktionsschicht wurde ein Filterpapier (25 SS 81 von Schleicher und Schüll) mit einer Lösung von

20 mmol/l L-γ-Glutamyl-3-carboxyl-4-nitroanilid und

100 mmol/l Glycylglycin in

100 mmol/l Trispuffer, pH 8,25

getränkt, getrocknet und wie die Abtrennschicht in 10 × 30 mm große Rechtecke geschnitten.

Zur Herstellung eines Systems zur Bestimmung von γ-GT im Vollblut wurde auf eine durchsichtige Kunststoffolie jeweils ein Rechteck aus imprägniertem Filterpapier und eine Abtrennschicht aufgebracht. Auf die Oberseite der Abtrennschicht wurden an einem Ende des Steifens 50 μl Vollblut mit jeweils verschiedener γ-GT-Aktivität aufgegeben und nach 15 Sekunden die Trennschicht auf die Filterpapierschicht so aufgedrückt, daß das Filterpapier unterhalb der Blutaufgabestelle gleichmäßig benetzt wurde. Anschließend wurde von der Unterseite des Systems durch die Kunststoffolie hindurch der Reaktionsablauf beobachtet. Innerhalb von 3 Minuten bildete sich in dem von der Flüssigkeit benetzten Bereich eine Gelbfärbung, die derjenigen entsprach, die man erhielt, wenn man anstelle des Blutes abzentrifugiertes Plasma des gleichen Blutes einsetzte.

## Patentansprüche

1. Verfahren zur Abtrennung von Plasma oder Serum aus Vollblut durch Aufbringen der Vollblutprobe auf eine aus einer saugfähigen, mit Lektin imprägnierten Matrix bestehende Abtrennschicht, dadurch gekennzeichnet, daß man nach einer vorgegebenen Zeit durch Anwendung eines Drucks das gebildete Plasma oder Serum in weitere, mit Reagenzien imprägnierte Schichten überführt, wobei das Volumen der zu trennenden Vollblutprobe maximal 50% des Saugvolumens der Abtrennschicht beträgt.

2. Verfahren nach Anspruch 1, dadurch gekennzeichnet, daß das Volumen der zu trennenden Vollblutprobe 20 bis 30% des Saugvolumens der Abtrennschicht beträgt.

3. Verfahren nach den Ansprüchen 1 und 2, dadurch gekennzeichnet, daß die die Abtrennschicht bildende Matrix aus einem Textilgewebe oder einem zellulosehaltigen Material besteht.

4. Verfahren nach den Ansprüchen 1 bis 3, dadurch gekennzeichnet, daß die Abtrennschicht die oberste Schicht eines mehrere Schichten enthaltenden Testsystems ist.

5. Verfahren nach den Ansprüchen 1 bis 4, dadurch gekennzeichnet, daß anschließend an die Abtrennschicht mindestens eine weitere, mit für eine Nachweisreaktion erforderlichen Substanzen imprägnierte Reaktionsschicht angeordnet ist.

6. Verfahren nach den Ansprüchen 1 bis 5, dadurch gekennzeichnet, daß zwischen der Abtrennschicht und der Raktionsschicht eine Zwischenschicht aus einer hydrophobierten Textilmatrix oder einem hydrophoben Netzwerk eingefügt ist.

6

# EP 0 194 502 B1

**Revendications**

1. Procédé pour la séparation du plasma ou du sérum de sang entier par application de l'échantillon de sang entier sur une couche de séparation constituée d'une matrice absorbante, imprégnée de lectine, caractérisé en ce que l'on transfère, après un temps prédéterminé, par application d'une pression, le plasma ou le sérum formé dans d'autres couches imprégnées par des réactifs, le volume de l'échantillon de sang entier à séparer représentant 50% au plus du volume absorbant de la couche de séparation.

2. Procédé selon la revendication 1, caractérisé en ce que le volume de l'échantillon de sang entier à séparer représente 20 à 30% du volume absorbant de la couche de séparation.

3. Procédé selon les revendications 1 et 2, caractérisé en ce que la couche de séparation formant la matrice est constituée d'un tissu textile ou d'un matériau cellulosique.

4. Procédé selon les revendications 1 à 3, caractérisé en ce que la couche de séparation constitue la couche supérieure d'un système de test comportant plusieurs couches.

5. Procédé selon les revendications 1 à 4, caractérisé en ce que, à la suite de la couche de séparation, est disposée au moins une autre couche réactive imprégnée des substances nécessaires pour une réaction de détection.

6. Procédé selon les revendications 1 à 5, caractérisé en ce que l'on insère, entre la couche de séparation et la couche réactive, une couche intermédiaire constituée d'une matrice textile rendue hydrophobe ou d'une structure réticulée hydrophobe.

**Claims**

1. Method for the separation of plasma or serum out of whole blood, by the sample of whole blood being applied to a separating layer consisting of an absorbent matrix which is impregnated with lectin, characterised in that, after a preset time, the plasma or serum which has formed is transferred, by application of a pressure, into further layers impregnated with reagents, the volume of the sample of whole blood which is to be separated being a maximum of 50% of the volume absorbable by the separating layer.

2. Method according to Claim 1, characterised in that the volume of sample of whole blood which is to be separated is 20 to 30% of the volume absorbable by the separating layer.

3. Method according to Claims 1 and 2, characterised in that the matrix forming the separating layer consists of a textile fabric or a cellulose-containing material.

4. Method according to Claims 1 to 3, characterised in that the separating layer is the uppermost layer of a test system containing several layers.

5. Method according to Claims 1 to 4, characterised in that located next to the separating layer is at least one further reaction layer impregnated with substances necessary for a detection reaction.

6. Method according to Claims 1 to 5, characterised in that an interlayer composed of a textile matrix which has been rendered water-repellent or of a hydrophobic network is inserted between the separating layer and the reaction layer.

7

EP  0 194 502  B1

Fig. 1

3
1
2

Fig. 2

3
5
4
1
2

Fig. 3

3
4
2
1